# EUROPEAN PATENT SPECIFICATION

(11) **EP 0 475 971 B1**
(45) Date of publication and mention of the grant of the patent: **13.04.1994**
(21) Application number: 90908306.5
(22) Date of filing: 09.05.1990
(51) Int. Cl.: A61K 37/14, C07K 15/22, C08H 1/02, C07F 9/58

(54) **A HEMOGLOBIN COMPOSITION AND ITS USE**
HÄMOGLOBINZUSAMMENSETZUNG UND IHRE VERWENDUNG
COMPOSITION D'HEMOGLOBINE ET SON UTILISATION

(30) Priority: 10.05.1989 NL 8901174
(43) Date of publication of application: 25.03.1992
(73) Proprietor: STAAT DER NEDERLANDEN, DE MINISTER VAN DEFENSIE, voor deze: HET HOOFD VAN DE AFDELING MILITAIR GENEESKUNDIG BELEID, 2511 CR 's-Gravenhage (NL)
(72) Inventor: BERBERS, Wilhelmus Antonius Maria, NL-3583 AL Utrecht (NL); BIESSELS, Petrus Theodorus Maria, NL-2342 AC Oegstgeest (NL); BLEEKER, Willem Karel, NL-1017 EV Amsterdam (NL); BAKKER, Joachim Cornelis, NL-1541 VX Koog aan de Zaan (NL)
(74) Representative: Smulders, Theodorus A.H.J., Ir.
(86) International application number: NL9000067
(87) International publication number: WO9013309

(56) References cited:
- EP-A- 0 361 719
- EP-A- 0 361 720
- WO-A-87/07832
- Journal of Laboratory and Clinical Medicine, voll. 113, no. 2, Febr. 1989, W.K. Bleeker et al.:"In vivo distribution and elimination of hemoglobin modified by intramolecular cross-linking with 2-nor-2-formylpyridoxal 5'-phosphate", see pages 151-161
- Biochemical and Biophysical Research Communications, vol. 156, no. 1, 14. Oct. 1988, Academic Press, Inc., R.E. Benesch et al.: "Bis-pyridoxal polyphosphates: A new class of specific intramolecular crosslinking agents for hemoglobin", see pages 9-14

## Description

### Background of the invention

Hemoglobin solutions have potential application in (wounded) man as oxygen-carrying plasma expanders in those situations in which the filling of the vascular system and the oxygen-transporting capacity of the blood are insufficient. The advantage over erythrocytes or blood is that the solutions are universally applicable without typing blood groups. A second advantage is that the hemoglobin solutions have a longer storage life. For this purpose, however, it is necessary to prepare and modify hemoglobin from erythrocytes in such a manner that the three main problems of hemoglobin solutions are minimized. These three problems are:
1) the presence of residues of membrane Fragments (stroma) originating from erythrocytes
2) the high intrinsic oxygen affinity outside the environment of the erythrocyte
3) the short retention time in the circulation because (dissociated) hemoglobin disappears rapidly from the vascular system because of leakage through the kidneys.
to 1) As early as 1967 Rabiner described a preparation of hemoglobin solutions in which stroma was removed by filtration and centrifugation so that earlier observed coagulation problems in the kidneys of dogs could be avoided (J. Expl. Med. 126, 1127-1142 (1967)). This method of removing stroma later proved insufficient to prevent every side effect.
to 2) In 1972 Benesch et al. (Biochemistry 11, 3576-3582 (1972)) described that a marked decrease of the oxygen affinity can be realized by covalently binding pyridoxal 5-phosphate (PLP) to hemoglobin. This permanent binding prevents PLP from leaving the free hemoglobin infused into the circulation, as would be the case with 2,3-di-phosphoglycerate (2,3-DPG). PLP can be bound at different sites to one of the β-globin chains in the 2,3-DPG binding cavity.
to 3) By polymerizing hemoglobin the retention time in the circulation can be prolonged. This is described by Mazur et al. (US patent 3,925,344 (1975)) with (m)ethyl glutarimidate, (m)ethyl succinimidate and (m)ethyl suberimidate, etc. and by Bonsen et al. (US patents 4,001,200, 4,001,401, 4,053,590 and 4,061,736 (1977)), inter alia with different dialdehydes, particularly glutaraldehyde. A problem of these modifications of hemoglobin is that the oxygen affinity substantially increases so that the oxygen release in vivo to the tissues cannot be optimal. By both coupling hemoglobin to PLP and polymerizing hemoglobin with glutaraldehyde Bonhard et al. (US patents 4,136,093 and 4,336,248 (1979)) have obtained an (only slight) improvement in the oxygen affinity in combination with a prolonged retention time. Rausch et al. (WO 88/03408) describe polymerization of bovine hemoglobin of which a long circulation time in test animals has been shown. The difference in species, however, will stand in the way of administration to human beings.

In spite of all these improvements clinical application was not yet relied on, since administration of small amounts of hemoglobin solution to healthy volunteers showed toxic effects (Savitsky et al., Clin. Pharmacol. Ther. 23, 73-90 (1978)). Recently, however, the group of Moss administered a comparable dose to healthy volunteers with no side effects being observed. The hemoglobin solution then used was a polymerization product of HbPLP analogous to the product according to Bonhard but improved as described in WO 87/07832 (Sehgal et al.). According to this patent unconverted hemoglobin must be removed from the polymerization mixture (to prevent negative effects on the renal function), which is achieved by ultrafiltration, gel filtration, and affinity chromatography.

Another approach to prolonging the retention time in the circulation and decreasing the oxygen affinity is intramolecular cross-linking. Thus dissociation of tetrameric Hb into dimers and leakage through the kidneys are prevented. That cross-linking of the β-chains prolongs the retention time was first shown by Bunn et al. with bis(N-maleidomethyl)ether (J. Exp. Med. 129, 909-934 (1969)). This modification, however, led to a considerable increase in the oxygen affinity. Bakker et al. (Adv. Exp. Med. Biol. 180, 345-356 (1985)) showed that by coupling with 2-nor-2-formylpyridoxal 5-phosphate (NFPLP; see formula 1 of the sheet of formulae), as described by Benesch et al.
(Biochem. Biophys. Res. Commun. 63, 1123-1129 (1975)), both a longer retention time and a lower oxygen affinity are realized. Walder et al. use diaspirin compounds for coupling α-globin chains (US patents 4,598,604 and 4,600,531 (1986)). The oxygen affinity of their coupling product is equal to that of erythrocytes and the retention time in the circulation is prolonged by a factor of 3. Bucci et al. (US patent 4,584,130 (1986)) couple hemoglobin intramolecularly to negatively charged organic reagents. Kavanaugh et al. use the bifunctional reagent 4,4'-diisothiocyanostilbene-2,2'-disulfonate (Biochemistry 27, 1804-1808 (1988)). Their coupling product has a physiologic oxygen affinity but is obtained in low yield.

The modification closest to the physiologic situation is the coupling with NFPLP or later mentioned analogous compounds. This led to coupling of the β-chains through valine-1 of the first β-chain and lysine-87 of the second β-chain (Arnone et al., J. Mol. Biol. 115, 627-642 (1977)). The NFPLP is covalently bound and is permanently present at the site where 2,3-DPG in the erythrocytes influences the oxygen affinity. This β-chain coupling was first described by Benesch et al. in 1975 and elaborated further by Van der Plas et al. (J. Lab. Clin. Med. 108, 253-260 (1986, Transfusion 27, 425-430 (1987), Transfusion 28, 525-530 (1988)). They found a substantially decreased Oxygen affinity, which guarantees a proper oxygen release under the most frequent conditions. The retention time in the circulation is prolonged by a factor of 3 (Bleeker et al., J. Lab. Clin. Med. 108, 448-455 (1986)). As a plasma expander with oxygen-transporting capacity, however, the cross-linked hemoglobin (HbNFPLP) is open to criticism for the following reasons:
1) the half-life period in the circulation of man will be about 8 hours, whereas 24 hours is desirable, and
2) the oxygen-transporting capacity cannot be more than half of that of normal blood, because the free hemoglobin can only be present in a concentration of maximally 6-7 g/100 ml in the circulation in view of the colloid-osmotic pressure.

According to the invention it has now been found that these problems can be removed by polymerizing the HbNFPLP. The invention combines a large number of earlier mentioned improvements. Instead of NFPLP, bis-pyridoxal polyphosphate compounds of formula 2 of the sheet of formulae, in which R is an oxygen atom, an orthophosphate group, a pyrophosphate group, a diphosphate residue, or a diphosphonate residue, as recently described by Benesch and Kwong (Biochem. Biophys. Res. Commun. 156, 9-14 (1988)) have proved useful as a starting material for the polymerization product described. The modified hemoglobin according to the invention is briefly indicated by poly HbXlβ, "Hb" indicating hemoglobin, "Xlβ" an intramolecular cross-linking through the β-chains (e.g., by means of-NFPLP or by means of bis-pyridoxal polyphosphates), and "poly" indicating that the intramolecularly cross-linked hemoglobin is polymerized.

### Detailed description of the invention

The product of this invention is a blood substitute consisting of a cross-linked, polymerized human hemoglobin solution. Human hemoglobin is a tetramer built up of two identical α-polypeptide chains and two identical β-chains which are non-covalently bound to each other. The tetramer can dissociate rapidly into two α,β-dimers. Dissociation into α,α-and β,β-dimers or into α- and β-monomers does not take place under physiologic conditions. To prevent that dissociation into α,β-dimers and thus leakage through the kidneys, the hemoglobin is first modified in such a manner that it comprises a covalent, intramolecular cross-link between the β-chains. The modification is effected by means of a reaction of deoxygenated hemoglobin with a water-soluble bifunctional reagent, as the earlier mentioned NFPLP or one of the bis-pyridoxal polyphosphate compounds. After having purified the thus modified hemoglobin with an ion exchange column, it not only shows an increased retention time in the circulation but also a substantially decreased oxygen affinity. By subsequently polymerizing the modified hemoglobin the oxygen affinity is corrected again to an almost physiologic value. Moreover, the retention time in the circulation of the cross-linked polymerized hemoglobin (polyHbXlβ) substantially increases. The polymerization is effected with a polymerizing reagent, preferably glutaraldehyde. During this process the molecular weight distribution is analyzed by means of gel permeation chromatography. The successive steps in the process of production will now be discussed in more detail.

The starting material for the production of the invention here described is fresh human blood screened for hepatitis B and HIV. Erythrocytes are isolated therefrom by removing the plasma by centrifugation, the leucocytes by adsorption to a leucocyte filter (Cellselect, NPBI), and the thrombocytes by removing the "buffycoat", after which the erythrocytes are washed three times with 0.9% NaCl. The purified sterile erythrocytes are the starting material for the process proper which is carried out as a whole between 2°C and 10°C, with the exception of the coupling which is effected at room temperature. At the end of each individual step of the process of production the modified or unmodified hemoglobin is always sterilized by filtration over a sterile 0.2 µm filter (Millipore).

The erythrocytes can be lysed by adding at least two volumes of distilled water. For separating the hemoglobin and the remainders of the red cells (stroma) cross-flow ultraflltration is applied in a Pellicon system (Millipore). Preferably, however, the erythrocytes are dialyzed on a similar Pellicon system against a hypotonic phosphate-buffered salt solution, resulting in that the red cells swell to the extent that the hemoglobin can leak to the outside. Since in this procedure the erythrocytes remain substantially intact, the thus obtained hemoglobin solution will contain fewer cell debris (membrane fragments, phospholipids).

The synthesis of the reagents ensuring the specific intramolecular coupling of the hemoglobin is an organochemical procedure which in the case of NFPLP consists of five steps, the starting material being pyridoxal hydrochloride. In the first step the N atom of this molecule is oxidized, and in the second step the oxygen is rearranged to the adjacent methyl group. In the third step the thus obtained alcohol is oxidized to an aldehyde. In the fourth step the two aldehyde groups are coupled with toluidine to the 2-nor-2-formylpyridoxal bistoluidine. This gives protection of the aldehyde groups in the last fifth step of the synthesis, the phosphorylation of the methyl alcohol group of the molecule with polyphosphoric acid. After separation of the toluidine groups by hydrolysis the resulting NFPLP is separated from inorganic phosphate by means of a cation exchange column.

For the synthesis of bispyridoxal polyphosphates first the tetrabutylammonium salt (TBA) of PLP is reacted with diphenylphosphochloridate (DPPC, Aldrich). To the crude reaction product is added the TBA salt of the desired phosphate. The reaction mixture is separated on an anion exchange column (Dowexl-X8®) with a gradient from 0 to 0.2 molar LiCl in a 0.01 molar HCl solution as an eluent.

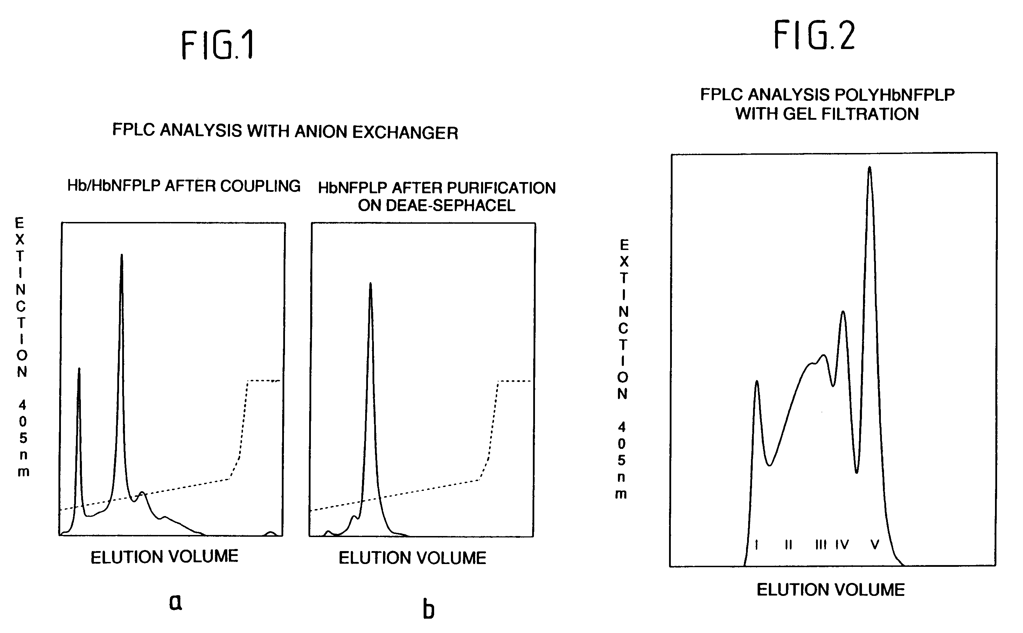

The stroma-free hemoglobin is coupled to NFPLP or a bispyridoxal polyphosphate compound in a molar ratio varying from 1:1 to 1:2. The coupling reaction takes place in a trishydrochloride buffer having a final concentration of about 0.1 molar and a pH of about 7.0. The hemoglobin solution is first deoxygenated completely, which may be done in a rotating flask into which nitrogen or another inert gas is introduced. Preferably, however, a gas exchanger is used (Endotronics), with the hemoglobin being circulated through flexible tubes that are substantially impermeable to oxygen (Tygon). By adding an excess of a reducing agent, preferably sodium borohydride (Merck), to the coupling mixture, the cross-link becomes irreversible and the binding covalent. A typical example of an analysis of a coupling mixture by means of ion exchange chromatography (Mono Q, Pharmacia) is shown in Fig. 1a. The coupled hemoglobin is dialyzed on the Pellicon system against a trishydrochloride buffer with a concentration of about 0.1 molar and a pH of about 8.3 to remove the excess of reagents and to create the right conditions for the next step in the process, the purification.

The coupled hemoglobin (HbXlβ) is purified with ion exchange chromatography. On a column filled with anion exchanger of the tertiary amine type, preferably diethylaminoethyl-A50, diethylaminoethyl-Sephacel or diethylaminoethyl-Sepharose (Pharmacia) HbXlβ is separated from uncoupled hemoglobin by means of a linear salt gradient with which the column is eluted. The salt gradient consists of an increasing concentration of sodium chloride from 0 to about 0.1 molar in the above tris-buffer with a pH of 8.3. Detection of hemoglobin is effected spectrophotometrically in a flow cuvette. The purified HbXlβ is collected separately from the other eluate by means of an automatically controlled valve (Pharmacia). A typical example of an analysis of the purified HbXlβ is shown in Fig. 1b. This purification step results in a specific product, in which there also occurs a substantial reduction of possibly toxic substances, such as membrane fragments of the erythrocyte, endotoxin, and viral antigens, as far as these are present in the stroma-free hemoglobin.

Subsequently, the purified HbXlβ is polymerized with preferably glutaraldehyde (Sigma) which is done by means of a kidney dialysis filter (Andante, Organon Tecknika). Such a filter consists of a large number of "hollow fibers" of a semipermeable membrane through which small molecules, such as water, salts, and also glutaraldehyde, can migrate freely to and fro, which large molecules, such as hemoglobin, cannot. Thus it is rendered possible to control the velocity of the polymerization reaction, because the glutaraldehyde can be added to the hemoglobin gradually and in small portions. The reaction time and the amount of added glutaraldehyde depends on the amount of HbXlβ and the desired molecular weight distribution of the final product. The polymerization results in a mixture of products that can be analyzed by gel permeation chromatography (Superose 12, Pharmacia). By such an analysis method the mixture can be separated into monomers, dimers, trimers, tetramers, and polymers. The ratio of these different polymers in the final product depends on the reaction time and the reaction velocity. Thus the mixture may vary in the following ratios (percentages of the total):

| polymers | tetra/trimers | dimers | monomers |
|---|---|---|---|
| 0 | 0 | 20 | 80 |
| 0 | 40 | 20 | 40 |
| 20 | 40 | 15 | 25 |
| 40 | 30 | 10 | 20 |

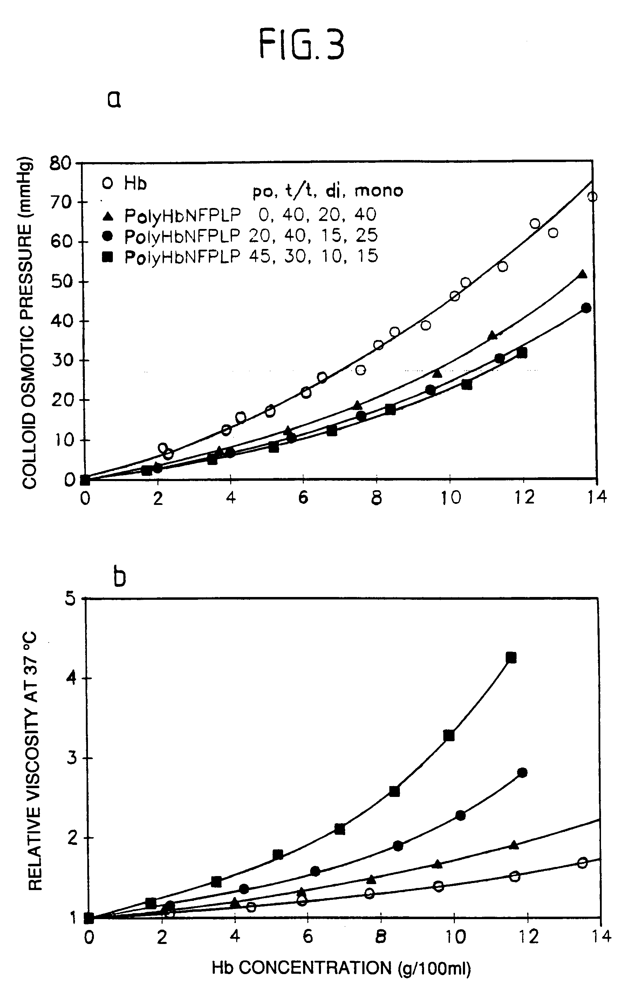

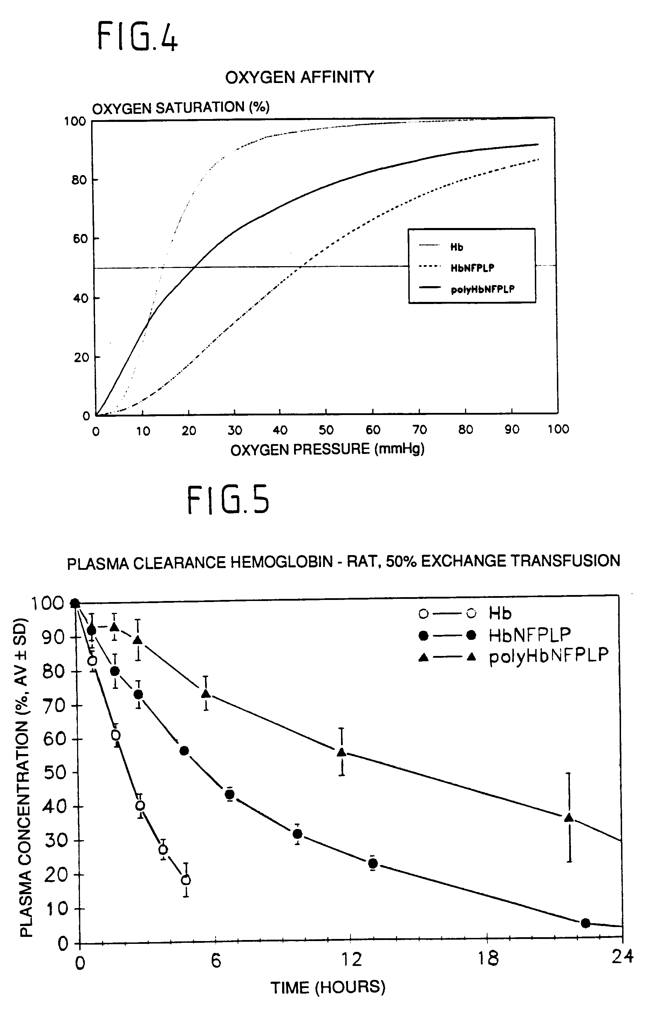

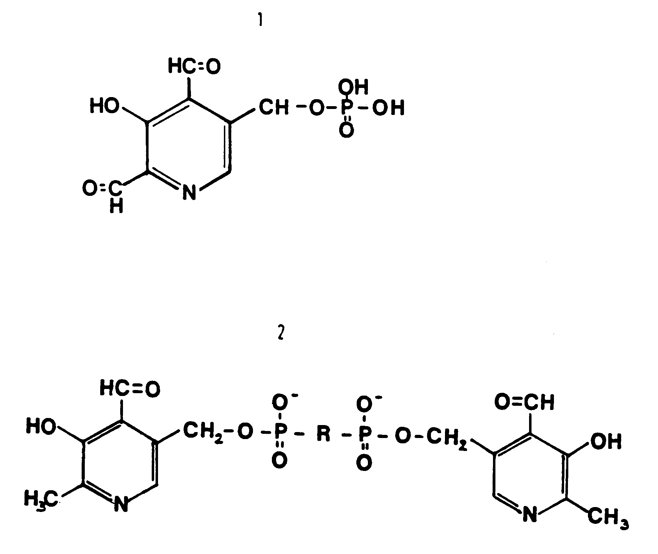

Finally, the whole mixture will consist of large polymers, unless the reaction is stopped in time. This may be done by adding an excess of lysine to the reaction mixture so that the free aldehyde groups still present are blocked and the polymerization stops. By adding a reducing reagent, preferably sodium borohydride, the cross-links become covalent. Thus a stable final product can be obtained. When determining the optimum degree of polymerization of the final product (polyHbXlβ), consideration must be given to the retention time in the circulation, but also to the effects of the polymerization on the colloid osmotic pressure and viscosity of polyHbXlβ. At an equal Hb concentration the colloid osmotic pressure will decrease with an increasing degree of polymerization, but the viscosity will increase. The hemoglobin may be polymerized to a mixture of which 40%, preferably at least 50%, consists of di-, tri-, and tetramers. Up to 40% of the hemoglobin may be present as monomer, but essentially no dissociable monomer is present. Fig 2 shows a typical example of a gel filtration pattern of polyHbXlβ. Of the final product, preferably 10% consists of polymers, 40% of tetra/trimers, 20% of dimers, and 30% of monomers. Such a degree of polymerization ensures that the retention time of polyHbXlβ in the circulation of rats is prolonged by a factor of about 7 when compared with unmodified hemoglobin. The colloid osmotic pressure is then reduced to such an extent that under physiologic conditions a concentration of 10 g/100 ml is possible and the viscosity only slightly increases to a value comparable to that of plasma (see Figs. 3 and 4). The molecular weight of monomer Hb is 68 kD; the different polymers are multiples thereof. The polymer fraction elutes with the void volume of the column which is used by routine for the gel filtration analyses. Analyses on columns with another range in separating capacity have shown that the molecular weight of the polymer fraction of polyHbXlβ ranges from 300 to 600 kD. The average molecular weight of the final product can be derived from the ratio of the different polymers in polyHbXlβ and will approximately range from 160 to 270 kD, particularly from 160 to 220 kD, although the average molecular weight may vary from 140 to 380 kD. Consequently, the average polymer in polyHbXlβ has a size ranging between dimer and trimer. The monomer fraction in the final product is 30%, but need not be removed therefrom, since the hemoglobin is coupled intramolecularly and is consequently not nephrotoxic.

The hemoglobin composition of the invention may have an iso-oncotic hemoglobin concentration of more than 9 g/100 ml ; a relative viscosity below 4, preferably below 2.5 ; an oxygen affinity corresponding to a p50 value of 19-24 mm Hg; and may have the form of a pharmaceutically acceptable solution or the form of a freeze dried composition.

### Advantages of the invention

The invention here described has the following advantages over existing products:
1) A first advantage concerns the intermediate product HbXlβ. This is obtained by specific coupling of an aldehyde to both β-chains of Hb. HbXlβ is formed with a yield of at least 70% and can be separated from unconverted nephrotoxic Hb by a single purification step with an ion exchanger. Coupling of Hb to PLP gives 4-6 products of which only 25% main product.
2) PolyHbXlβ is a mixture of polymers which also comprises non-polymerized HbXlβ. An important advantage is that this non-polymerized HbXlβ need not be removed. In fact, HbXlβ cannot dissociate into dimers through the intramolecular cross-link so that it is not filtered through the kidney and does not cause, e.g., nephrotoxicity. This is contrary to, e.g., polyHbPLP the non-polymerized HbPLP of which is nephrotoxic so that it is necessary to remove this carefully by means of complicated purification steps. This fact enables a simpler process of producing polyHbNFPLP and consequently a higher efficiency.
3) The oxygen affinity of polyHbXlβ is lower than that of polyHbPLP (Sehgal) so that in vivo a better oxygen release must be possible.
4) The preparation is started from human erythrocyte suspensions purified from leucocytes, thrombocytes and plasma proteins by filtration and centrifugation so that contamination with metabolites and other substances from these fractions is avoided.
5) The stroma removal from the hemoglobin solutions is measured by means of the phospholipid content and the membrane antigen glycophorin-α. The phospholipid content is lower than 4 µg/g Hb (erythrocytes: 9 mg/g Hb). The amount of glycophorin-α is less than 0.01% of what is present in erythrocytes.
6) PolyHbXlβ can be prepared in the following ratio: 30% monomer, 20% dimer, 40% tri/tetramer and 10% polymer. Then the said solution has an iso-oncotic hemoglobin concentration of 10 g/100 ml and a relative viscosity lower than 2 with respect to water. For polyHbPLP Bonhard described that a hemoglobin content of maximally 8 g/100 ml can be reached, whereas the product of Sehgal already has a viscosity of 3 cp at 8 g/100 ml.
7) The disintegration of HbNFPLP is already properly understood, which is important to the evaluation of possible side effects. No accumulation of HbNFPLP has been found to occur in liver or kidneys (Bleeker et al., J. Lab. Clin. Med. 113, 151-171 (1989)). It has been shown that the coupling molecule, NFPLP, is substantially excreted in urine and feces within 8 days.
8) A number of measures and steps taken in the process of production result in that contamination with viruses, if any, is almost impossible: inter alia, by screening donors, using leucocyte filtration and ion exchange chromatography of the coupling mixture.

### Examples

The invention will be explained with reference to the following examples. It is important to recognize, however, that these examples are given for illustrative purposes and elucidate the reproducibility of the invention, but in no way mean the definitive conditions of the different steps of the process of production and comprise the complete scope of the invention. All operations are carried out under sterile conditions (GMP standards) and all the steps of production are carried out at about 5°C and under atmospheric pressure, with the exception of the coupling reaction which is carried out at room temperature.

### Example 1

### PolyHbNFPLP

### A. Preparation of a stroma-free hemoglobin solution.

Fresh human blood is released from plasma by centrifugation, from leucocytes through a leucocyte filter and from thrombocytes by removal of the buffy-coat. The units of red blood cells are washed three times in sterile plastic bags with 0.5 l 0.9% NaCl and centrifuged for 5 minutes at 3200 rpm. The washed erythrocytes ("packed cells") of 8 units are pooled and suspended in 4 volumes of a 0.1 molar NaCl solution buffered with 0.01 molar phosphate, which results in a 6-8 l suspension with a hematocrite of 20-30%. On the Pellicon system provided with a 0.45 µm cut-off HVLP cassette (Millipore) this suspension is dialyzed first against about 5 l of the phosphate buffered 0.1 molar NaCl solution and then against a phosphate buffered 0.05 molar NaCl solution. When the hemoglobin begins to leak from the cells, the filtrate is collected. Usually about 15 to 20 l of filtrate is collected and then concentrated on the same Pellicon system but now provided with a 10,000 dalton cut-off PTGC cassette. The ultrafiltrate has a hemoglobin concentration of 10 to 20 g/100 ml and is sterilized by filtration. The yield of stroma-free hemoglobin solution is 80-90%, and the methemoglobin content is below 1%. The phospholipid content in the hemoglobin solution is below 4µg/g Hb.

### B. Synthesis of NFPLP

This is a 5-step synthesis starting from pyridoxal hydrochloride (Merck).

### 1. The synthesis of the N oxide.

a. 200 g of pyridoxal hydrochloride is dissolved in 2 l of ethanol (96%) and boiled. After 1 hour of refluxing the solution is cooled, and an equivalent of sodium bicarbonate (84 g) is added cautiously. The solution is refluxed for 30 minutes. The salt precipitate is filtered over "Celite Hyflo-Super-Cel" (hyflo, Merck) and rinsed with a small amount of ethanol (96%). The ethanolic solution is further used for reaction b.
b. The solution of the pyridoxal ethyl acetal is cooled to -30°C with CO₂/acetone. For half an hour a solution of 345 g of methachloroperbenzoic acid (Aldrich) in 1 l of ethanol (-10°C) is added with stirring. The light-yellow reaction mixture is allowed to stand for 40 hours at -30°C. The N oxide crystallizes out into white crystals, is filtered and washed with ether.

### 2. The rearrangement of the N oxide.

a. 35 g of N oxide and 100 g of trifluoroacetic anhydride (Aldrich) are cooled to -30°C. The trifluoroacetic acid is dropped slowly to the solid N oxide, and the suspension is stirred for 12-24 hours at room temperature. Then the dark red solution is hydrolyzed with 40 ml 2 molar HCl and refluxed for 1 hour.
b. After evaporation the residue is dissolved in a minimum amount of methanol, and tetrahydrofuran (THF) is added until the solution becomes turbid. Crystallization occurs overnight at-30°C.

### 3. The oxidation with pyrolusite.

In a mixture of 50 ml of methanol and 200 ml of THF, 6 g of the HCl salt is dissolved, 25 g of activated manganese dioxide (Merck) is added, and the mixture is stirred for 12-24 hours at room temperature. The reaction is followed by thin-layer chromatography (TLC, Merck) with an eluent of chloroform/methanol (95/5). The pyrolusite and the resulting manganese chloride are filtered over hyflo and washed with methanol. Silica gel is added to the filtrate and then evaporated. The silica gel is eluted with successively 800 ml of chloroform and 500 ml of chloroform/methanol 97.5/2.5. The solution is evaporated.

### 4. The preparation of 2-nor-2-formylpyridoxal bistoluidine.

In boiling water, 4 g of 2-nor-2-formylpyridoxal is hydrolyzed to the free dialdehyde. The reaction is followed by TLC. The solution is poured slowly into a solution of 6.6 g of p-toluidine (Merck) in 100 ml of methanol and is refluxed until turbidity of the solution. After cooling to room temperature the solution is allowed to stand overnight at 4°C. The orange precipitate formed is filtered and washed with cold dry ether.

### 5. Phosphorylation.

Polyphosphoric acid (PPA) is made from a mixture of 10 g of P₂O₅ and 13 g of H₃PO₄. 15 ml of PPA is added to 1.5 g of bistoluidine. The reaction mixture is stirred for 1 hour at 60°C, shut off from the air. Then 7.5 ml of 0.1 N HCl is added. This reaction mixture is stirred for 15 minutes at 60°C. The dark purple mixture is applied to a column, filled with a cation exchanger (Biorad 50Wx80; H form, 200-300 mesh) and eluted with distilled water. The eluate is collected in 10 ml fractions.

With the Molybdate test (W.C. Hulsman, thesis of 1958, Amsterdam) there is measured which fractions contain free phosphoric acid. The fractions containing organic phosphate (A₄₁₅ in 1M phosphate buffer (pH=7) in 1:100 dilution > 0.1 absorption unit) are pooled. The NFPLP is stored as a pool at 4°C in the dark.

### C. Coupling of NFPLP to the stroma-free hemoglobin

The stroma-free hemoglobin solution is dialyzed on the Pellicon system against 10 volumes of 0.1 molar trishydrochloride solution with a pH of 7.0. Usually about 1 l of 10 g/100 ml hemoglobin is coupled, but the volume can be easily scaled up. The hemoglobin solution having a pH varying from 6.9 to 7.1 is deoxygenated by means of a membrane gas exchanger through which nitrogen gas is passed at a rate of 2 l/min. The hemoglobin solution is pumped through the gas exchanger from a closed reservoir at a rate of 0.5 l/min. (Sarns pump). Within 1 hour the oxygen is removed, which is monitored by means of an oxygen saturation measurement (below 5%, measured by multicomponent analysis on a diode array spectrophotometer (Hewlett-Packard)). If desired, the hemoglobin can also be deoxygenated in a closed rotating flask having a volume of 10 l into which nitrogen is introduced. This procedure gives the same result as regards the content of deoxyHb, but takes three to five times longer.

To the deoxygenated hemoglobin solution in the reservoir a deoxygenated aqueous solution of NFPLP is added slowly. The molar ratio relative to hemoglobin may vary from 1:1 to 2:1. Mostly, a small excess of NFPLP is added. The reaction time is at least 1 hour. Then a 20-fold excess of sodium borohydride is added to the coupling mixture. The sodium borohydride is dissolved in 30-50 ml of a 0.001 molar KOH solution which is also deoxygenated. Since nitrogen can also be introduced into the reservoir, the gas formation occurring during reduction causes no problem and is therefore discharged together with the nitrogen. At the end of the reaction the excess of reagents is removed by dialysis and the coupling mixture is sterilized by filtration. The maximum yield of coupled hemoglobin is 60-70%, which can be determined with the integration of the peaks of a chromatogram obtained in an FPLC analysis of the mixture on an ion exchange column (see Fig. 1a). With increasing salt concentration first Hb and then HbNFPLP is eluted from the column.

### D. Purification of HbNFPLP

At the end of the reaction the coupling mixture is dialyzed against 10 volumes of the starting buffer of the ion exchange chromatography, a 0.1 molar tris-hydrochloride buffer having a pH of 8.3. A column of 25 to 5 cm is filled with diethylaminoethyl Sephacel in starting buffer and eluted with at least 5 column volumes of that starting buffer at a rate of 2 ml/min. (Cenco pump). Subsequently, 200 ml of the coupling mixture of maximally 10 g/100 ml is applied to the column and then eluted with at least 1 column volume of starting buffer. The column is then eluted with a gradient of 0-0.1 molar NaCl in about 3 l of starting buffer. Analysis of the different fractions takes place spectrophotometrically by means of a flow cuvette at 540 nm. The different fractions (uncoupled Hb and HbNFPLP) can be collected by means of an automatically controlled valve apart from the other eluate. The purified HbNFPLP is concentrated on the Pellicon system (PTGC cassette) and sterilized by filtration. Analysis of the purified product takes place by FPLC ion exchange chromatography (see Fig. 1b). More than 95% of the thus obtained HbNFPLP is pure. After this column purification the yield of HbNFPLP is 40-50% of the coupling mixture, which corresponds to 70-80% of the coupled product.

### E. Polymerization of HbNFPLP

The purified HbNFPLP is polymerized by means of a kidney dialysis filter. The HbNFPLP solution is pumped through the artificial kidney from a reservoir at a rate of 0.3 l/min., while as dialysate phosphate buffered NaCl solution (PBS) is circulated at a rate of 0.5 l/min (Sarns pumps). To the dialysate 1 ml of 25% glutaraldehyde is always added stepwise, and the velocity of the polymerization reaction is monitored by means of gel filtration analyses. The amount of glutaraldehyde required to obtain a certain degree of polymerization depends on the amount and concentration of the employed HbNFPLP solution. A typical example of a gel filtration analysis of a final product is shown in Fig. 2. In this case the final product consists of 10% polymer, 40% tetra/trimer, 20% dimer, and 30% monomer, which is the desired ratio, but in principle the degree of polymerization depends on the reaction time. Starting from 0.5 l of 5 g/100 ml HbNFPLP at least 5 ml of 25% glutaraldehyde is required to reach such a final stage as shown in Fig. 2 within a reasonable time (a few hours). Per 1 mole of hemoglobin at least 8 moles of glutaraldehyde are added. The reaction can be stopped by adding to the reservoir a 10-fold excess, relative to glutaraldehyde, of 1 molar lysine solution with a pH of about 7.0. Also, the dialysate is pumped out and replaced by new PBS. Then the dialysis is continued for some hours, and by replacing the dialysate a number of times the polyHbNFPLP can be introduced into any desired buffer, for exchange transfusions preferably in kidney dialysis buffer. Sometimes the thus produced polyHbNFPLP slowly polymerizes further in the months to follow, which is preferably avoided by also adding to the polymerization mixture, after the polymerization reaction with lysine has stopped, an amount of sodium borohydride equimolar with respect to glutaraldehyde so as to give a stable final product. After dialysis the polyHbNFPLP may be concentrated and sterilized by filtration. The yield of the polymerization reaction is almost 100% and the methemoglobin content in the final product is below 5% (multicomponent analysis). The phospholipid content is determined by extracting phospholipids from the polyHbNFPLP solution by means of chloroform and methanol. The extract is treated with perchloric acid and the released phosphate forms a complex with ammonium molybdate and malachite green (Merck), the absorption of which is measured at 630 nm. The content in the final product is below 1 µg/g Hb, which means a reduction by a factor of 4 relative to the stroma-free hemoglobin solution. The amount of glycophorin-α in the final product is below the detection limit of the determination for this erythrocyte membrane antigen (radioimmunoassay with monoclonal antibody), which reflects a value belower 0.01% of what is present in erythrocytes. The endotoxin content is determined with the Limulus test (Bleeker et al., in Progress in Clinical and Biological Research, vol. 189: Bacterial Endotoxins, 293-302 (1985), eds. ten Cate et al.) and is below 2 EU/g Hb for polyHbNFPLP.

### F. Biophysical characterization of polyHbNFPLP

Polymerization influences some properties of the hemoglobin solution which are essential in case of use as a blood substitute: the colloid osmotic pressure, the viscosity, and the oxygen affinity.

### 1. Colloid osmotic pressure

When the polymerization proceeds further, the colloid osmotic pressure which may be caused by the solution decreases. Fig. 3a shows the relation between the concentration and the colloid osmotic pressure for different hemoglobin solutions. The colloid osmotic pressure is determined with a pressure transducer (Statham P23Db) over an ultrafilter membrane. The concentration is determined photometrically. The horizontal dotted line indicates the colloid osmotic pressure of human plasma. Thus it can be seen that the iso-oncotic concentration (i.e. the concentration at which the solution has the same colloid osmotic pressure as plasma) of unmodified hemoglobin (Hb) is 7 g/100 ml. By polymerization the iso-oncotic concentration is increased to about 9 g/100 ml for polyHbNFPLP without large polymers and to about 11 g/100 ml for polyHbNFPLP in which 45% larger polymers are formed. This higher iso-oncotic concentration means that during transfusion a higher plasma concentration can be obtained without the risk of overfilling the vascular system.

### 2. Viscosity

Fig. 3b shows the relation between concentration and viscosity for the same hemoglobin solutions. The relative viscosity is measured with an Ostwald viscosimeter. This graph shows the other side of the polymerization: the increase in the viscosity. At a low degree of polymerization (polyHbNFPLP 0,40,20,40) this increase, however, is still so low that the relative viscosity at 9 g/100 ml is 1.5 and is therefore still below that of plasma. At a higher degree of polymerization (polyHbNFPLP 45,30,10,15) a relative viscosity of 4 has been found at 11 g/100 ml, i.e. a value equal to that for whole blood. This means that the viscosity of polyHbNFPLP remains acceptable if polymerization is not carried out beyond 45% polymers (molecular weight ranging from 300 to 600 kD).

### 3. Oxygen affinity

The oxygen-binding properties have been examined with an oxygen dissociation curve analyzer (DCA, Radiometer), under physiologic conditions of pH, pCO₂_{,} and temperature. Fig. 4 shows the measurements of unmodified hemoglobin (Hb), HbNFPLP and polyHbNFPLP. The 50% saturation point, the p50 of polyHbNFPLP is at a pO₂ of 22 mm Hg and is thus substantially equal to the p50 of whole blood (26 mm Hg). A clear improvement over the unmodified hemoglobin solution (p50:14 mm Hg) and HbNFPLP (p50:45 mm Hg) has taken place.

### G. In vivo characteristics of polyHbNFPLP

### 1. Retention time in circulation

The retention time in the circulation has been examined in rats after replacement of 50% of the blood by a hemoglobin solution. Fig. 5 shows the plasma disappearance curve of polyHbNFPLP with 30-40% polymers, as compared with that of an unmodified hemoglobin solution (Hb) and a hemoglobin solution modified by only intramolecular cross-linking with NFPLP. The moment the plasme concentration is halved (T50%) is at about 2 hours for Hb. Intramolecular cross-linking improves the T50% by a factor of 3. After polymerization the T50% has increased by a factor of 7 with respect to Hb. In view of literature data on research with different hemoglobin solutions in different test animals including apes, a retention time of more than 24 hours is expected for polyHbNFPLP in human beings.

### 2. Toxicity to the kidney

After replacement of 50% of the blood volume by a hemoglobin solution in rats 40% of the administered dose is excreted in the urine. Histochemical research shows a strong accumulation of hemoglobin in the kidneys, a clear indication of a limited renal function. After intramolecular cross-linking of the β-chains less than 5% of the dose is excreted in the urine, histochemical research only showing a minimum accumulation of HbNFPLP. The occurrence of an impairment of the kidney function caused by HbXlβ is therefore improbable. After an exchange transfusion with polyHbNFPLP hemoglobin is not detectable in the urine, nor in the kidneys themselves. Thus an impairment of the kidney function caused by intrarenal precipitates of polyHbNFPLP is therefore impossible.

### Example 2

### polyHb(bisPL)P₂

The preparation of polyHb(bisPL)P₂ proceeds identically with that of polyHbNFPLP, as described in Example 1, with the exception of the synthesis of the coupling molecule. The characteristics of the final product (polyHb(bisPL)P₂) are also substantially identical with that of polyHbNFPLP. The synthesis of (bisPL)P₂ proceeds as follows:

After reaction of 0.002 moles of PLP with 0.0025 mole of DPPC, 0.004 mole of PLP-TBA in 10 ml of dry pyridine is added thereto. After the reaction mixture has been allowed to stand for 1 night, it is applied to an ion exchange column and eluted with 0.01 molar HCl. (BisPL)P₂ comes from the column as first fraction, followed by PLP. After neutralization the fraction is evaporated; yield 350 mg product (30%). UV spectrum: maxima at 294 and 335 nm in 0.1 molar HCl, 330(s) and 391 nm in phosphate buffer with a pH of 7, 308 and 391 nm in 0.1 molar NaOH.

### Description of the Figures

### Fig. 1

Chromatograms obtained with an anion exchange column (Mono Q). Left-hand chromatogram (Fig. 1a): mixture of Hb (left-hand peak) and HbNFPLP (right-hand peak) obtained after the coupling reaction of Hb with NFPLP. Right-hand chromatogram (Fig. 1b): purified main product (HbNFPLP) from the coupling mixture.

### Fig. 2

Polymer composition of polyHbNFPLP, determined by gel filtration over a Superose 12 column. The elution pattern shows the following peaks: I - polymers larger than tetramer II - tetramers, III - trimers, IV - dimers, V - monomers.

### Fig. 3

Upper graph (Fig. 3a): relation between concentration and colloid osmotic pressure of an unmodified hemoglobin solution (Hb) and three differently polymerized solutions of polyHbNFPLP. Of the polyHbNFPLP solutions the polymer composition is indicated in percentages (po: polymers, t/t: tetra/trimers. di: dimers, mo: monomers). The horizontal dotted line indicates the colloid osmotic pressure of human plasma.

Lower graph (Fig. 3B): relation between concentration and viscosity of the same hemoglobin solutions.

### Fig. 4

Oxygen dissociation curves, measured under physiologic conditions of pH, pCO₂, and temperature, of an unmodified hemoglobin solution (Hb), HbNFPLP, and polyHbNFPLP.

### Fig. 5

Plasma disappearance curves of free hemoglobin in rats after replacement of 50% of the blood volume by: 1 - unmodified hemoglobin (Hb, n=5), 2 - HbNFPLP (n=3), and 3-polyHbNFPLP (n=3).

## Claims

1. An essentially stroma-free hemoglobin composition comprising a polymerized human hemoglobin cross-linked intramolecularly through the β-chains.

2. A hemoglobin composition according to claim 1, in which the hemoglobin is cross-linked intramolecularly through the β-chains by means of a pyridoxal 5-phosphate derivative with 2 aldehyde groups.

3. A hemoglobin composition according to claim 1, in which the hemoglobin is cross-linked intramolecularly through the β-chains by means of 2-nor-2-formylpyridoxal 5-phosphate.

4. A hemoglobin composition according to claim 1, in which the hemoglobin is cross-linked intramolecularly through the β-chains by means of a bis-pyridoxal polyphosphate of formula 2, in which R is an oxygen atom, an orthophosphate group, a pyrophosphate group, a diphosphate residue, or a diphosphonate residue.

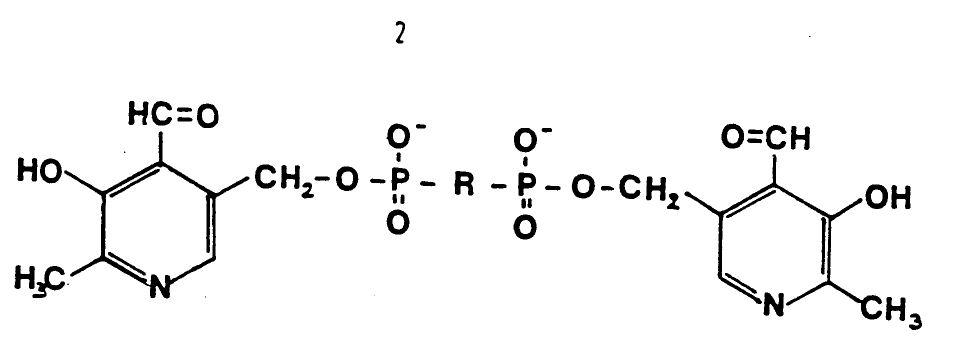

5. A hemoglobin composition according to claim 1, in which the hemoglobin is polymerized by means of glutaraldehyde.

6. A hemoglobin composition according to claim 1, in which the hemoglobin is polymerized to an average molecular weight ranging from 140 to 380 kD, preferably from 160 to 270 kD.

7. A hemoglobin composition according to claim 1, in which the hemoglobin is polymerized to a mixture of which 40%, preferably at least 50%, consists of di-, tri-, and tetramers.

8. A hemoglobin composition according to claim 1, in which up to 40% of the hemoglobin is present as monomer, but essentially no dissociable monomer is present.

9. A hemoglobin composition according to claim 1, characterized by an iso-oncotic hemoglobin concentration of more than 9 g/100 ml and a relative viscosity below 4, preferably below 2.5.

10. A hemoglobin composition according to claim 1, characterized by an oxygen affinity satisfying a p50 value of 19-24 mm Hg.

11. A hemoglobin composition according to claim 1 in the form of a pharmaceutically acceptable solution or in the form of a freeze dried composition.

## Patentansprüche

1. Im wesentlichen stromafreie Hämoglobinzusammensetzung, die ein intramolekular über die β-Ketten vernetztes, polymerisiertes humanes Hämoglobin enthält.

2. Hämoglobinzusammensetzung nach Anspruch 1, in der das Hämoglobin intramolekular über die β-Ketten durch ein Pyridoxal-5-phosphat-Derivat mit 2 Aldehydgruppen vernetzt ist.

3. Hämoglobinzusammensetzung nach Anspruch 1, in der das Hämoglobin intramolekular über die β-Ketten durch 2-Nor-2-formylpyridoxal-5-phosphat vernetzt ist.

4. Hämoglobinzusammensetzung nach Anspruch 1, in der das Hämoglobin intramolekular über die β-Ketten durch ein Bispyridoxalpolyphosphat der Formel 2 vernetzt ist, in der R ein Sauerstoffatom, eine Orthophosphatgruppe, eine Pyrophosphatgruppe, einen Diphosphatrest oder einen Diphosphonatrest bedeutet.

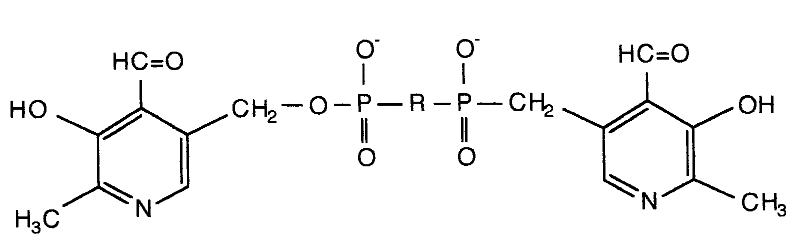

5. Hämoglobinzusammensetzung nach Anspruch 1, in der das Hämoglobin durch Glutaraldehyd polymerisiert ist.

6. Hämoglobinzusammensetzung nach Anspruch 1, in der das Hämoglobin bis zu einem Durchschnittsmolekulargewicht zwischen 140 und 380 kD, vorzugsweise zwischen 160 und 270 kD polymerisiert ist.

7. Hämoglobinzusammensetzung nach Anspruch 1, in der das Hämoglobin zu einem Gemisch polymerisiert ist, das zu mindestens 40%, vorzugsweise mindestens 50%, aus Di-, Tri- und Tetrameren besteht.

8. Hämoglobinzusammensetzung nach Anspruch 1, in der bis zu 40% des Hämoglobins als Monomer anwesend ist, aber im wesentlichen kein dissoziierbares Monomer anwesend ist.

9. Hämoglobinzusammensetzung nach Anspruch 1, gekennzeichnet durch eine isoonkotische Hämoglobinkonzentration von über 9 g/100 ml und eine relative Viskosität von unter 4, vorzugsweise unter 2,5.

10. Hämoglobinzusammensetzung nach Anspruch 1, gekennzeichnet durch eine Sauerstoffaffinität, die einem p50-Wert von 19-24 mm Hg entspricht.

11. Hämoglobinzusammensetzung nach Anspruch 1 in Form einer pharmazeutisch akzeptablen Lösung oder in Form einer gefriergetrockneten Zusammensetzung.

## Revendications

1. Préparation d'hémoglobine essentiellement dépourvue de stroma, comprenant une hémoglobine humaine polymérisée, réticulée à l'intérieur des molécules par l'intermédiaire des chaînes β.

2. Préparation d'hémoglobine selon la revendication 1, dans laquelle l'hémoglobine est réticulée à l'intérieur des molécules par l'intermédiaire des chaînes β, au moyen d'un dérivé de 5-phosphate de pyridoxal comportant deux groupes aldéhyde.

3. Préparation d'hémoglobine selon la revendication 1, dans laquelle l'hémoglobine est réticulée à l'intérieur des molécules par l'intermédiaire des chaînes β, au moyen de 5-phosphate de 2-nor-2-£ormylpyridoxal.

4. Préparation d'hémoglobine selon la revendication 1, dans laquelle l'hémoglobine est réticulée à l'intérieur des molécules par l'intermédiaire des chaînes β, au moyen d'un polyphosphate de bis-pyridoxal selon la formule 2, dans laquelle R est un atome d'oxygène, un groupe orthophosphate, un groupe pyrophosphate, un résidu de diphosphate ou un résidu de diphosphonate.

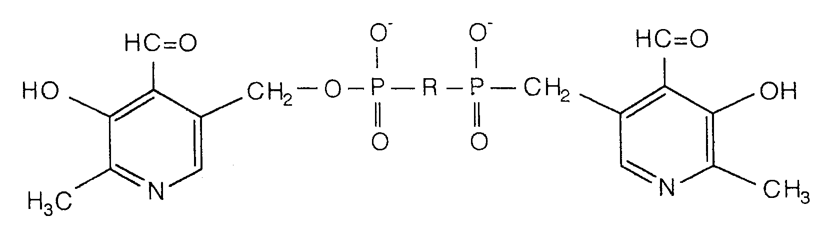

5. Préparation d'hémoglobine selon la revendication 1, dans laquelle l'hémoglobine est polymérisée au moyen de glutaraldéhyde.

6. Préparation d'hémoglobine selon la revendication 1, dans laquelle l'hémoglobine est polymérisée jusqu'à un poids moléculaire moyen de 140 à 380 kD, de préférence de 160 à 270 kD.

7. Préparation d'hémoglobine selon la revendication 1, dans laquelle l'hémoglobine est polymérisée en un mélange dont 40 %, de préférence au moins 50 %, sont constitués de dimères, de trimères et de tétramères.

8. Préparation d'hémoglobine selon la revendication 1, dans laquelle jusqu'à 40 % de l'hémoglobine sont présents sous forme de monomère, mais dans laquelle essentiellement aucun monomère dissociable n'est présent.

9. Préparation d'hémoglobine selon la revendication 1, caractérisée par une concentration en hémoglobine isooncotique supérieure à 9 g/100 ml et par une viscosité relative inférieure à 4, de préférence inférieure à 2,5.

10. Préparation d'hémoglobine selon la revendication 1, caractérisée par une affinité oxygène vérifiant une valeur du p50 de 19-24 mm Hg.

11. Préparation d'hémoglobine selon la revendication 1, présentant la forme d'une solution utilisable en pharmacie ou celle d'une préparation lyophilisée.
